(19) Europäisches Patentamt
European Patent Office
Office européen des brevets

(11) Publication number: **0 057 870**
**B1**

(12) **EUROPEAN PATENT SPECIFICATION**

(45) Date of publication of patent specification: **31.07.85**

(21) Application number: **82100606.1**

(22) Date of filing: **02.10.79**

(60) Publication number of the earlier application in accordance with Art. 76 EPC: **0 009 800**

(51) Int. Cl.[4]: **C 07 C 87/62,** C 07 C 87/50, C 07 C 93/14, A 61 K 31/135 // C07C131/00, C07C87/28, C07C103/375, C07C103/76, C07C119/08, C07C103/44, C07C103/737, C07C103/38

# (54) N-optionelly substituted-2-amino-alpha-phenylphenethylamines and a method for their preparation.

(30) Priority: **05.10.78 US 948896**

(43) Date of publication of application: **18.08.82 Bulletin 82/33**

(45) Publication of the grant of the patent: **31.07.85 Bulletin 85/31**

(84) Designated Contracting States: **AT BE CH DE FR GB IT NL SE**

(56) References cited:
**FR-A-1 298 310**
**GB-A-1 218 135**

**CHEMICAL ABSTRACTS, vol. 54, no. 18, 25th September 1960, column 18427 e-i, Columbus Ohio (USA); YUTAKA YAMAKAWA: "III Substituted 1,2-diphenylethylamine derivatives"**

(73) Proprietor: **HOECHST AKTIENGESELLSCHAFT**
**Postfach 80 03 20**
**D-6230 Frankfurt am Main 80 (DE)**

(72) Inventor: **Martin, Lawrence Leo**
**No. 3, Box 81 Concord Road**
**Lebanon, New Jersey (US)**
Inventor: **Worm, Manfred, Dr.**
**27 Erbsenacker**
**D-6200 Wiesbaden (DE)**
Inventor: **Crichlow, Charles Anthony**
**892 Davidson Road**
**Piscataway New Jersey 08854 (US)**

**EP 0 057 870 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel N-alkyl-2-amino-α-phenylphenethylamines and to a method of their preparation.

The compounds of this invention have the formula I

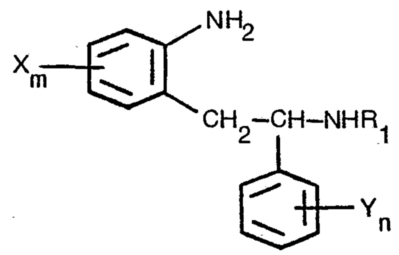

II

in which $R_1$ is hydrogen, alkyl of from 1 to 3 carbon atoms, cycloalkyl-$C_1$—$C_3$-alkyl wherein the cycloalkyl radical has 3 to 6 carbon atoms, or aralkyl having up to 8 carbon atoms, X and Y are the same or different and each can be chlorine, bromine, fluorine, alkoxy or alkyl each of from 1 to 3 carbon atoms, hydroxy, or trifluoromethyl; m is the integer 0, 1 or 2;, and n is the integer 0, 1, 2 or 3. The compounds of the present invention can be prepared according to the following sequence of reactions in which R, $R_1$, X, Y, m and n are as previously defined, unless otherwise indicated.

a) A 2-(2-nitrophenyl)acetophenone of the formula III

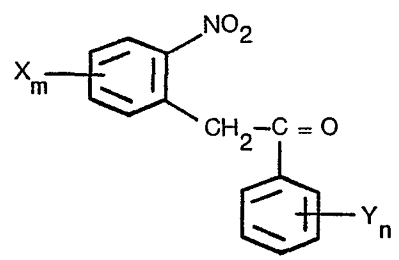

III

is converted to an oxime of the formula IV

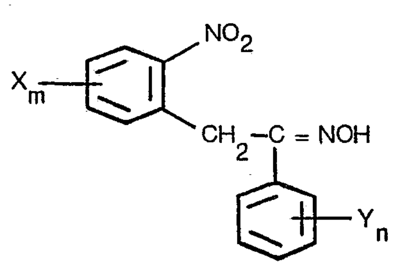

IV

by any convenient method known to the art. A preferred method involves refluxing the acetophenone in a mixture of ethyl alcohol, aqueous sodium acetate and hydroxylamine.

b) A compound of formula IV is acylated with acetic anhydride to provide the corresponding oxime acetate of the formula

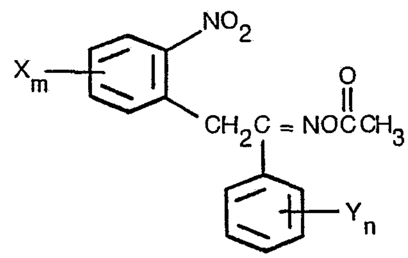

V

c) A compound of formula V is carefully reduced to provide the corresponding 2-nitro-a-phenylphenethylamine of the formula VI

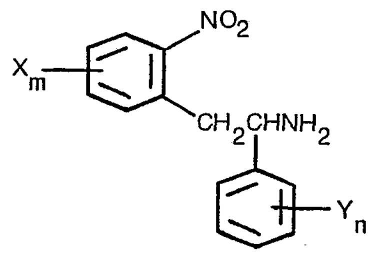

VI

The reducing agent used in this step must be compatible with the phenyl nitro group. Diborane is preferred. Additionally, this reduction is carried out in the presence of a solvent such as tetrahydrofuran and at a low temperature of from about 0°C to about 30°C or at ambient temperature.

d) A compound of formula VI is further reduced to provide the corresponding 2-amino-α-phenylphenethylamine, a novel compound of the present invention, of the formula IIa

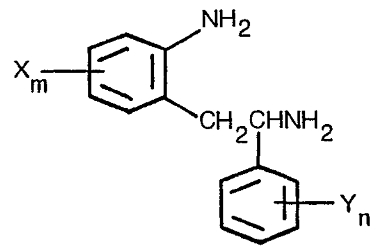

IIa

A preferred reducing method involves hydrogenation with a palladium on carbon catalyst or Adam's catalyst. Also other chemical reducing methods are suitable such as the use of tin and hydrochloric acid.

e) A compound of formula VI is converted by any method known to the art, to the corresponding amide of the formula

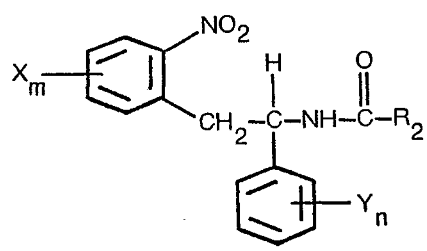

VII

wherein $R_2$ is hydrogen, alkyl, cycloalkylalkyl or aralkyl. This conversion is carried out with an appropriate carboxylic acid anhydride or halide in the presence of a suitable solvent. Additionally, when $R_2$ is hydrogen, a preferred method is the mixed anhydride procedure utilizing a mixture of acetic anhydride and formic acid at a temperature of from about 0°C to 100°C with a suitable solvent such as benzene.

f) A compound of formula VII is reduced in a manner consistent with the procedure of step c, above, to provide the corresponding N-alkyl-α-phenyl-2-nitrophenylethylamine of the formula

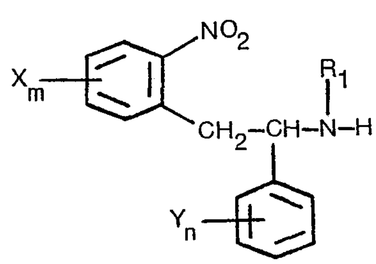

VIII

in which $R_1$ is alkyl.

g) A compound of the formula VIII is reduced in a manner consistent with the procedure of step d, above, to provide the corresponding N-alkyl-2-amino-α-phenylphenethylamine, of the formula II.

Compounds depicted in formula III are either generally available or prepared as described below. O. Hromatka et al., Monatsh, *100,* (1969) describe the following synthesis in which X is chlorine or bromine:

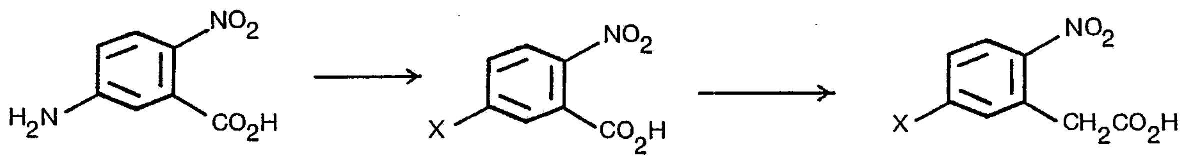

Routine manipulation of this synthesis would provide corresponding compounds in which X is fluorine, hydroxy, methoxy or cyano.

Additionally, 2,4-dinitroaniline can be treated by routine methods to ultimately provide a compound of the formula

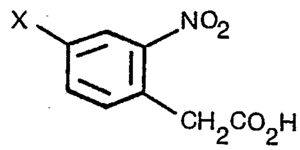

through the following precursors:

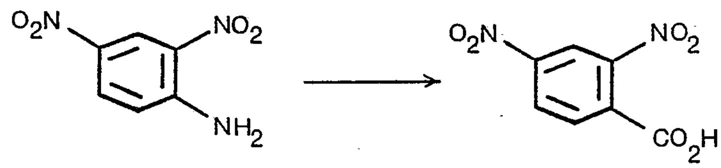

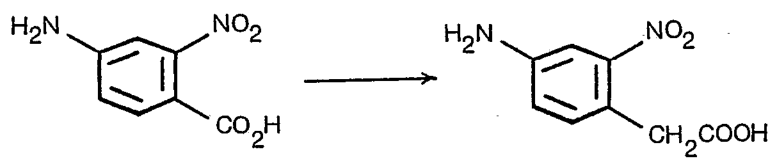

Thereafter, compounds of formula III can be prepared by Friedel-Crafts acylation as follows:

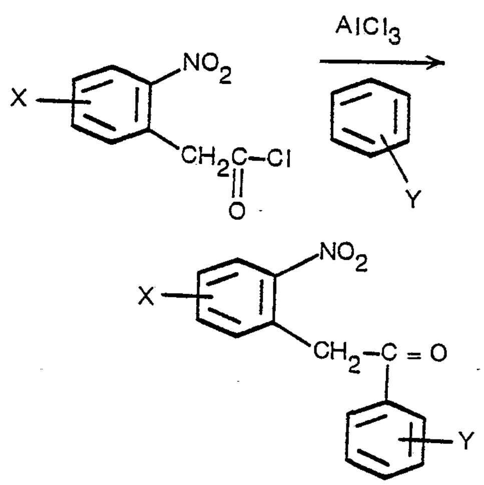


The novel compounds of the formula I may also be used as intermediate compounds for the preparation of compounds of the formula I

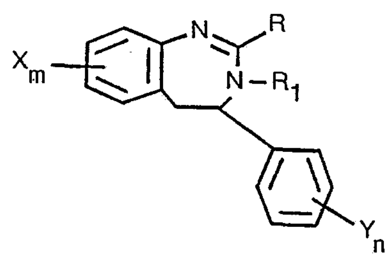

wherein X, Y, $R_1$, m and n are as defined above. These compounds are useful as antidepressants, analgetics

and anticonvulsants and can be prepared by cyclization of the compounds of the formula II in the presence of an acid catalyst and triethyl orthoformate as described in copending European Patent Application publication No. 7800.

Example 1

a. A stirring mixture of 43.0 g of 2-(2-nitrophenyl)-acetophenone, 200 ml of 95% ethyl alcohol, 31.2 g of sodium acetate and 24.3 g of hydroxylamine hydrochloride in 100 ml of water is refluxed for 1 hour and then permitted to stand for 16 hours. Thereafter, the precipitate is collected by suction filtration and the filter cake is sequentially washed with 100 ml of 60% aqueous ethyl alcohol, two 100 ml portions of water, dried and recrystallized from 95% ethyl alcohol to provide nearly colorless crystals, mp 119—121°C, of 2-(2-nitrophenyl)-acetophenone oxime. [The 2-(2-nitrophenyl)acetophenone starting material is reported in Chem., Absts., *63,* 16237g (1965)].

b. A stirring solution of 5.0 g of 2-(2-nitrophenyl)-acetophenone oxime in 10 ml of potassium hydroxide dried pyridine is treated with 5 ml of acetic anhydride in three equal portions. After total addition, the solution is heated on a steam bath for 45 minutes with the exclusion of moisture and then decanted into 50 ml of ice water. An oil separates which solidifies with continued stirring. The solid is successively collected by vacuum filtration, washed with water, dried under vacuum over potassium hydroxide pellets and recrystallized from 95% ethyl alcohol to provide nearly colorless crystals, mp 63—66°C, of 2-(2-nitrophenyl)-acetophenone oxime acetate.

c. 150 ml of a 1M solution of diborane in tetrahydrofuran are added to a stirring mixture of 9.0 g of 2-(2-nitrophenyl)-acetophenone oxime acetate and 150 ml of tetrahydrofuran over a 20 minute span with ice-water bath cooling. After total addition, the reaction mixture is stirred for 48 hours at ambient temperature before carefully adding, with stirring, 150 ml of a 5% hydrochloric acid solution. After this total addition the tetrahydrofuran is distilled under reduced pressure and the residue is extracted thrice with 200 ml portions of ether. The aqueous phase is basified with 50% sodium hydroxide and the alkaline phase is extracted thrice with 300 ml portions of ether. The combined ether extracts are dried and then concentrated, leaving an oil which, in ether, is converted to its hydrochloric acid salt. The salt is collected by vacuum filtration and then washed thoroughly with ether. The dried salt is recrystallized from methyl alcohol to provide colorless crystals, mp 261—263°C, dec., of 2-nitro-α-phenylphenethylamine hydrochloride.

d. 19.4 g of 25% (W/W) a sodium methoxide in methyl alcohol solution are added to a suspension of 19.6 g of 2-nitro-α-phenylphenethylamine hydrochloride in 100 ml of ethyl alcohol. Afte the addition, the mixture is stirred with steam bath warming before being filtered. The filter cake is washed with 70 ml of 95% ethyl alcohol and the filtrates are combined and then transferred into a 500 ml Parr hydrogenation bottle which had been charged with 2.0 g of a 10% palladium/carbon catalyst and 20 ml of 95% ethyl alcohol. The mixture is hydrogenated for 3 hours at 50 psig at ambient temperature. Thereafter, the mixture is suction filtered and the filtrate is evaporated, leaving an orange oil which is dissolved in 100 ml of chloroform. The chloroform solution and the washing is extracted twice with 100 ml portions of chloroform. All the chloroform solutions are combined and then sequentially dried, filtered and the filtrate is evaporated, leaving an orange oil which is diluted with 12 ml of cyclohexane. This solution is seeded with a crystalline product obtained from the previously obtained crude oil by crystallization of a sample from a small amount of cyclohexane, stirred vigorously and permitted to stand at 5°C. for 48 hours. Thereafter, the mother liquor is decanted and the crystalline precipitate is pulverized and collected in a Buchner funnel. The filter cake is washed with cyclohexane, dried and recrystallized from cyclohexane to provide colorless crystals, mp 43—44°C., of 2-aminophenylphenethylamine.

Example 2

a. A mixture of 27.6 g of acetic anhydride and 16.7 ml of formic acid is heated at 50—67°C for 15 minutes and then cooled to 10—15°C. A solution of 31.8 g of 2-nitro-α-phenylphenethylamine, free base of Example 1c, in benzene is added at such a rate as to maintain the reaction mixture's temperature at 10—15°C. After total addition, the suspension is stirred for 48 hours at 50°C and then stirred for an additional 48 hours at ambient temperature. Thereafter, the material is collected by suction filtration and then rinsed well with ether. The filtrate is evaporated under reduced pressure and the resulting solid is combined with the filter cake. The combined solid is dried under vacuum at 40°C for 2 hours and the dried material is dissolved in methylene chloride and this solution is successively washed with 5% hydrochloric acid solution, washed with water, dried, filtered and evaporated leaving a solid. The solid is recrystallized from ethyl alcohol to provide, after drying under vacuum at 90°C., light yellow crystals, mp 151—153°C., of N-formyl-2-nitro-α-phenylphenethylamine.

b. A solution of 96 ml of 1M diborane in tetrahydrofuran and 50 ml of tetrahydrofuran is added dropwise to an ice cold suspension of 13 g of N-formyl-2-nitro-α-phenylphenethylamine in 150 ml of tetrahydrofuran. After the effervescence ceases, 28 ml of 5N hydrochloric acid are added dropwise.

Thereafter, the solvent is removed under reduced pressure and the resulting oily mixture is basified with a 50% sodium hydroxide aqueous solution. The basified mixture is extracted with several portions of ether and the combined ether extracts are successively dried, filtered and evaporated, providing an oil. The oil is dissolved in benzene and this solution is treated with ethereal hydrochloric acid. The solvent is removed and the resulting material is recrystallized from isopropyl alcohol to provide, after drying over

xylene for 24 hours, light yellow crystals, mp 192—196°C., of N-methyl-2-nitro-α-phenylphenethylamine hydrochloride.

c. A solution of 0.5 g of N-methyl-2-nitro-α-phenylphenethylamine hydrochloride in 20 ml of ethyl alcohol is added to a Parr hydrogenation bottle previously charged with 0.2 g of platinum dioxide, Adam's catalyst, and 5 ml of ethyl alcohol. This is pressurized to 50 psi with hydrogen for 5 minutes, then to 15 psi and placed on a Parr shaker for 30 minutes. The mixture is filtered through a fine sintered glass funnel and the filtrate is evaporated. The resulting material is recrystallized from isopropyl alcohol to provide, after drying, colorless crystals, mp 190—191°C., of 2-amino-N-methyl-α-phenylphenethylamine hydrochloride.

Example 3

2-Amino-N-ethyl-α-phenylphenethylamine dihydrochloride (II)

3.00 g of N-Ethyl-2-nitro-α-phenylphenethylamine hydrochloride is treated with 100 ml of hot 95% ethanol containing 1.10 g of potassium hydroxide for 15 minutes, after which the mixture is filtered and placed in a Paar flask. 0.15 g of 10% palladium on charcoal is added and the mixture is hydrogenated at room temperature and 50 psia for 3 hours, after which the catalyst is removed. The ethanol is evaporated from the filtrate and the resulting yellow oil is poured onto 100 ml of water. The biphasic aqueous mixture is extracted with methylene chloride (2 × 100 ml). The combined methylene chloride extracts are dried over anhydrous magnesium sulfate and the methylene chloride evaporated to afford a colorless oil. The oil is dissolved in 50 ml of ether. Ethereal hydrogen chloride (100 ml) is added precipitating the hydrochloride acid salt as an oil. The ethanol is evaporated and the oil is dissolved in 20 ml of i-propanol with heating. Upon cooling, white crystals, mp >248°C dec, fell from solution providing 2-amino-N-ethyl-α-phenylphenethylamine dihydrochloride.

Analysis:

| | | | |
|---|---|---|---|
| Calculated for $C_{16}H_{20}N_2 \cdot 2HCl$: | 61.35%C; | 7.08%H; | 8.94%N. |
| Found: | 61.25%C; | 7.11%H; | 8.95%N. |

Example 4

2-Amino-α-phenyl-N-propylphenethylamine dihydrochloride (II)

3.34 g of 2-Nitro-α-phenyl-N-propylphenethylamine hydrochloride is heated in 100 ml of 95% ethanol containing 1.21 g of potassium hydroxide for 15 minutes, after which the precipitated sodium chloride is removed by filtration. The ethanolic filtrate is placed in a Parr flask containing 0.15 g of 10% palladium on charcoal and hydrogenated at 50 psia for 2 hours. The catalyst is then removed and the ethanol evaporated. The resulting yellow oil is poured onto 100 ml of water and the biphasic mixture is extracted with methylene chloride (2 × 100 ml). The combined methylene chloride extracts are dried over anhydrous magnesium sulfate and the methylene chloride evaporated affording a yellow oil. The oil is dissolved in 50 ml of ethanol. Ethereal hydrogen chloride (50 ml) is added, precipitating the hydrochloride salt. The ethanol is evaporated and the resulting white powder is recrystallized from 20 ml of i-propanol to give crystals, mp >243°C dec, of 2-amino-α-phenyl-N-propylphenethylamine dihydrochloride.

Analysis:

| | | | |
|---|---|---|---|
| Calculated for $C_{17}H_{24}N_2Cl_2$: | 62.39%C; | 7.39%H; | 8.56%N |
| Found: | 62.16%C; | 7.32%H; | 8.49%N. |

Example 5

2-Amino-N-cyclohexylmethylene-α-phenylphenethylamine dihydrochloride (II)

7.0 g of N-Cyclohexylmethylene-2-nitro-α-phenylphenethylamine hydrochloride is treated with 200 ml of warm 95% ethanol containing 2.07 of potassium hydroxide for 15 minutes, after which the mixture is filtered and placed in a Paar flask containing 0.35 g of 10% palladium on charcoal. The mixture is hydrogenated at 50 psia for 2 hours. After removal of the catalyst, the ethanol is evaporated and the resulting biphasic mixture is poured onto 200 ml of water. The aqueous mixture is extracted with methylene chloride (2 × 200 ml). The combined methylene chloride extracts are dried over anhydrous magnesium sulfate and the methylene chloride is evaporated giving a colorless oil. The oil is dissolved in 100 ml of ether. Ethereal hydrogen chloride (200 ml) is added and the salt precipitates from solution. The hydrochloride salt is collected as a gummy powder, which is recrystallized from i-propanol to provide crystals, mp 205°C dec, of 2-amino-N-cyclohexylmethylene-α-phenylphenethylamine dihydrochloride.

Analysis:

| | | | |
|---|---|---|---|
| Calculated for $C_{21}H_{28}N_2 \cdot 2HCl$: | 66.14%C; | 7.93%H; | 7.35%N. |
| Found: | 65.90%C; | 7.86%H; | 7.34%N. |

Example 6

2-Amino-N-methyl-α-(4-methylphenyl)phenethylamine dihydrochloride (II)

To a mixture of 10 g of α-(4-methylphenyl)-N-methyl-2-nitrophenethylamine and 150 ml of ethanol, 3.7 g of potassium hydroxide is added. The mixture is stirred for 15 minutes and the potassium chloride filtered

off. The solution is hydrogenated at room temperature in the presence of 0.5 g of 10% palladium on charcoal for 2 hours. The catalyst is filtered off and ethereal-hydrogen chloride is added to the ethanolic filtrate. The mixture is evaporated and the residue is recrystallized from ethanol-ether giving crystals, mp 225—228°C. of 2-amino-N-methyl-α-(4-methylphenyl)phenethylamine dihydrochloride.

Analysis:

| | | | |
|---|---|---|---|
| Calculated for $C_{16}H_{20}N_2 \cdot 2HCl$: | 61.35%C; | 7.08%H; | 8.91%N. |
| Found: | 61.18%C; | 7.02%H; | 8.87%N. |

Example 7

2-Amino-α-(4-methoxyphenyl)-N-methylphenethylamine dihydrochloride (II)

A mixture of 10.02 g of α-(4-methoxyphenyl)-N-methyl-2-nitrophenethylamine hydrochloride, 100 ml of dichloromethane and 100 ml of water is treated with excess 10% sodium hydroxide solution. The dried (over anhydrous sodium sulfate) organic phase is concentrated to afford 8.74 g of a yellow oil. A mixture of the oil, 100 ml of absolute ethanol and 0.5 g of 10% palladium on charcoal catalyst is hydrogenated at 50 psia and ambient temperature on a Parr apparatus. Filtration and concentration of the filtrate on a rotary evaporator affords 10.35 g of an oil. A solution of the oil and 20 ml of methanol is treated with excess ethereal hydrogen chloride. Further dilution with ether affords a gum which solidifies on trituration with fresh ether. Recrystallization from 25 ml of isopropanol gives crystals, mp 223—226°C dec, of 2-amino-α-(4-methoxyphenyl)-N-methylphenethylamine dihydrochloride.

Analysis:

| | | | |
|---|---|---|---|
| Calculated for $C_{16}H_{20}N_2O \cdot 2HCl$: | 58.37%C; | 6.74%H; | 8.51%N. |

Example 8

N-Acetyl-2-amino-α-(3,4-dimethoxyphenyl)-N-methylphenethylamine (II)

A solution of 3.8 g of N-acetyl-α-(3,4-dimethyloxyphenyl)-N-methyl-2-nitrophenethylamine in 100 ml of ethanol is hydrogenated at room temperature and 50 psia over 0.5 g of 10% palladium on charcoal for approximately 20 minutes. The catalyst is filtered and the solvent evaporated giving product. Recrystallization from 95% ethanol gave crystals, mp 139—142°C, of N-acetyl-2-amino-α-(3,4-dimethoxyphenyl)-N-methylphenethylamine.

Analysis:

| | | | |
|---|---|---|---|
| Calculated for $C_{19}H_{24}N_2O_3$: | 69.53%C; | 7.37%H; | 8.53%N. |
| Found: | 69.67%C; | 7.33%H; | 8.56%N. |

Example 9

1-(4-Fluoro-2-methylphenyl)-2-(2-nitrophenyl)ethanone (III)

To a mixture of 181 g of o-nitrophenylacetic acid in 400 ml of 1,2-dichloroethane, 78 ml of thionyl chloride is added dropwise at room temperature. The mixture is warmed to approximately 65°C then allowed to stand overnight at ambient temperature. To 400 ml of m-fluorotoluene, 133 g of aluminium chloride is added in portions followed by the dropwise addition of the acid chloride solution keeping the temperature approximately 20—25°C. The mixture is gradually warmed to 60°C and held at 60°C until gas evolution ceases. The reaction mixture is poured into concentrated hydrochloric acid and ice and allowed to stand over the weekend. Additional methylene chloride is added to the reaction mixture. The organic layer is separated, washed with 5% sodium hydroxide solution and water, dried over anhydrous sodium sulfate and filtered. Evaporation of the filtrate gives a black oil. The oil is extracted with boiling isopropyl ether which on cooling yields product. Recrystallization from hexane provides crystals, mp 72—74°C, of 1-(4-fluoro-2-methylphenyl)-2-(2-nitrophenyl)ethanone.

Analysis:

| | | | |
|---|---|---|---|
| Calculated for $C_{15}H_{12}FNO$: | 65.93%C; | 4.43%H; | 5.13%N. |
| Found: | 66.01%C; | 4.47%H; | 5.10%N. |

Example 10

1-(4-Chlorophenyl)-2-(2-nitrophenyl)ethanone (III)

A mixture of 46 g of α-(4-chlorobenzoyl)-β-dimethylamino-2-nitrostyrene, 150 ml of dioxane and 50 ml of water is heated under reflux for 18 hours. The solution is concentrated. The residue is extracted with methylene chloride, dried over anhydrous sodium sulfate and evaporated to an oil. The oil is dissolved in a small volume of 95% ethanol and the product crystallizes. Recrystallization from ethanol gives crystals, mp 70—72°C, of 1-(4-chlorophenyl)-2-(2-nitrophenyl)ethanone.

Analysis:

| | | | |
|---|---|---|---|
| Calculated for $C_{14}H_{10}ClNO_3$: | 61.00%C; | 3.66%H; | 5.08%N. |
| Found: | 60.96%C; | 3.68%H; | 5.15%N. |

Example 11

1-(4-Fluorophenyl)-2-(2-nitrophenyl)ethanone (III)

To a mixture of 10 g of o-nitrophenylacetic acid in 29 ml of fluorobenzene, 4.3 of thionyl chloride is added dropwise at room temperature. The mixture is warmed at 45°C for 3 1/2 hours. To the cooled solution 8.1 g of aluminum chloride is added in portions. During the addition there is a slight exothermic reaction and the temperature rose to 40°C. The mixture is warmed to 45°C for approximately 1 1/2 hours. The reaction mixture is poured into concentrated hydrochloric acid-ice and extracted with ether. The ether extract is washed with 5% sodium hydroxide solution, water, dried over anhydrous sodium sulfate and evaporated to give a crude solid. Recrystallization from 95% ethanol gives crystals, mp 80—81°C, of 1-(4-fluorophenyl)-2-(2-nitrophenyl)ethanone.

Analysis:

| | | | |
|---|---|---|---|
| Calculated for $C_{14}H_{10}FNO_3$: | 64.86%C; | 3.89%H; | 5.40%N. |
| Found: | 64.64%C; | 3.94%H; | 5.31%N. |

Example 12

1-(4-Methoxyphenyl)-2-(2-nitrophenyl)ethanone oxime acetate (V)

A stirred solution of 15.0 g of 1-(4-methoxyphenyl)-2-(2-nitrophenyl)ethanone oxime and 30 ml of potassium hydroxide dried pyridine is treated dropwise with 15.0 ml of acetic anhydride. After stirring overnight at ambient temperature with exclusion of moisture, the solution is heated for 2 hours on a steam bath. The solution is then decanted into 300 ml of ice water and the resultant biphasic mixture is extracted with a total of 270 ml of methylene chloride. The organic phase is washed once with dilute acetic acid (18 ml of glacial acetic acid diluted with 200 ml of water) and then twice with water. Concentrated of the dried (over anhydrous sodium sulfate) organic phase affords 21.0 g of yellow oil. A solution of the oil and 125 ml of ether is washed sequentially with 50 ml-portions each of 5% hydrochloric acid solution, water, 5% sodium bicarbonate solution and water. The (dried over anhydrous sodium sulfate) organic phase is concentrated to a cloudy yellow oil which is subjected to azeotropic distillation with absolute ethanol. On standing overnight at ambient temperature, small rosettes of crystals form and a rosette is removed for use as seed crystals. The remaining material is dissolved in 40 ml of 95% ethanol, cooled slowly until an oil begins to separate and seeded with the previously isolated crystals. The crystalline precipitate is collected by vacuum filtration, washed with 95% ethanol and dried *in vacuo* to provide crystals, mp 65—68.5°C, of 1-(4-methoxyphenyl)-2-(2-nitrophenyl)ethanone oxime acetate.

Analysis:

| | | |
|---|---|---|
| Calculated for $C_{17}H_{16}N_2O_5$: | 62.19%C; | 4.91%H. |
| Found: | 62.16%C; | 4.85%H. |

Example 13

1-(4-Methoxyphenyl)-2-(2-nitrophenyl)ethanone oxime (IV)

A stirred suspension of 46.0 g of 1-(4-methoxyphenyl)-2-(2-nitrophenyl)ethanone and 240 ml of 95% ethanol is treated with a solution of 22.6 g of hydroxylamine hydochloride, 46.3 g of sodium acetate trihydrate and 130 ml of water. The stirred suspension is heated to reflux and 95% ethanol (180 ml) is added. The mixture is heated for 3 hours under reflux whereupon a clear yellow-colored solution is obtained. After stirring overnight at ambient temperature, excess ethanol is removed on a rotary evaporator and the residue is diluted with approximately 1 l of water. An oil separates and crystallizes. The solid is collected, washed with water and dried at 40°C overnight *in vacuo.* Recrystallization from 100 ml of 95% ethanol provides crystals, mp 106—110°C, of 1-(4-methoxyphenyl)-2-(2-nitrophenyl)ethanone oxime.

Analysis:

| | | |
|---|---|---|
| Calculated for $C_{15}H_{14}N_2O_4$: | 62.93%C; | 4.93%H. |
| Found: | 62.95%C; | 4.93%H. |

Example 14

1-(4-Fluorophenyl)-2-(2-nitrophenyl)ethanone oxime acetate (V)

To a solution of 72.7 g of 1-(4-fluorophenyl)-2-(2-nitrophenyl)ethanone oxime in 175 ml of pyridine, 70 ml of acetic anhydride is added dropwise. The mixture is warmed on the steam bath for 1 1/2 hours. The reaction mixture is poured into ice water and the solution made slightly acid with hydrochloric acid. The material which precipitates is filtered, dried and recrystallized from 95% ethanol giving crystals, mp 74—75°C, of 1-(4-fluorophenyl)-2-(2-nitrophenyl)ethanone oxime acetate.

Analysis:

| | | | |
|---|---|---|---|
| Calculated for $C_{16}H_{13}FN_2O_4$: | 60.76%C; | 4.14%H; | 8.86%N. |
| Found: | 60.66%C; | 4.13%H; | 8.78%N. |

Example 15

1-(4-Fluorophenyl)-2-(2-nitrophenyl)ethanone oxime (IV)

To a solution of 6.62 g of 1-(4-fluorophenyl)-2-(2-nitrophenyl)ethanone in 40 ml of 95% ethanol, 3.55 g of hydroxylamine hydrochloride in 10 ml of water and 4.48 g of sodium acetate in 10 ml of water are added. The mixture is refluxed 3 hours and allowed to stand overnight. The ethanol is removed *in vacuo* and the product is extracted with ether, dried over anhydrous sodium sulfate and evaporated. Recrystallization from 95% ethanol gives crystals, mp 139—142°C, of 1-(4-fluorophenyl)-2-(2-nitrophenyl)ethanone oxime.

Analysis

| | | | |
|---|---|---|---|
| Calculated for $C_{14}H_{11}FN_2O_3$: | 61.31%C; | 4.04%H; | 10.21%N. |
| Found: | 61.15%C; | 4.08%H; | 10.27%N. |

Example 16

1-(4-Methylphenyl)-2-(2-nitrophenyl)ethanone oxime (IV)

To a mixture of 50 g of 1-(4-methylphenyl)-2-(2-nitrophenyl)ethanone in 200 ml of 95% ethanol, a solution of 27.8 g of hydroxylamine hydrochloride in 50 ml of water and a solution of 36.1 g of sodium acetate are added. The reaction mixture is refluxed for 3 hours and allowed to stand overnight at ambient temperature. The product which crystallizes from the reaction mixture is filtered and dried. Recrystallization from 95% ethanol provides crystals, mp 129—132°C, of 1-(4-methylphenyl)-2-(2-nitrophenyl)ethanone oxime.

Analysis:

| | | | |
|---|---|---|---|
| Calculated for $C_{15}H_{14}N_2O_3$: | 66.66%C; | 5.22%H; | 10.36%N. |
| Found: | 66.59%C; | 5.31%H; | 10.45%N. |

Example 17

1-(4-Methylphenyl)-2-(2-nitrophenyl)ethanone oxime acetate (V)

To a solution of 47.3 g of 1-(4-methylphenyl)-2-(2-nitrophenyl)ethanone oxime in 100 ml of pyridine, 40 ml of acetic anhydride is added dropwise. The mixture is warmed on the steam bath (internal temperature approximately 80°C) for 1 hour. The mixture is poured into water and the product which crystallizes is filtered giving crystals, mp 95—98°C, of 1-(4-methylphenyl)-2-(2-nitrophenyl)ethanone oxime acetate.

Analysis:

| | | | |
|---|---|---|---|
| Calculated for $C_{17}H_{16}N_2O_4$: | 65.38%C; | 5.20%H; | 8.97%N. |
| Found: | 65.42%C; | 5.16%H; | 9.00%N. |

Example 18

1-(3,4-Dimethoxyphenyl)-2-(2-nitrophenyl)ethanone oxime (IV)

A mixture of 154 g of 1-(3,4-dimethoxy)-2-(2-nitrophenyl)ethanone in 600 ml of 95% ethanol, 70.9 g of hydroxylamine hydrochloride in 150 ml of water and 92 g of sodium acetate in 150 ml of water is refluxed 7 hours. The product which crystallizes on standing overnight at room temperature is filtered and dried giving crystals, mp 129—130°C, of 1-(3,4-dimethoxyphenyl)-2-(2-nitrophenyl)ethanone oxime.

Analysis:

| | | | |
|---|---|---|---|
| Calculated for $C_{16}H_{16}N_2O_5$: | 60.75%C; | 5.10%H; | 8.85%N. |
| Found: | 60.75%C; | 5.15%H; | 8.84%N. |

Example 19

1-(3,4-Dimethoxyphenyl)-2-(2-nitrophenyl)ethanone oxime acetate (V)

A mixture of 156 g of 1-(3,4-dimethoxyphenyl)-2-(2-nitrophenyl)ethanone oxime and 100 g of acetic anhydride is warmed on a steam bath for 30 minutes. The product crystallizes on standing overnight at room temperature. Trituration with hexane gives crystals, mp 118—120°C, of 1-(3,4-dimethoxyphenyl)-2-(2-nitrophenyl)ethanone oxime acetate.

Analysis:

| | | | |
|---|---|---|---|
| Calculated for $C_{18}H_{18}N_2O_6$: | 60.37%C; | 5.06%H; | 7.82%N. |
| Found: | 60.14%C; | 5.11%H; | 7.78%N. |

Example 20

1-(2-Fluorophenyl)-2-(2-nitrophenyl)ethanone oxime (IV)

A mixture of 12.3 g of 2′-fluoro-2-(2-nitrophenyl)acetophenone in 80 ml of 95% ethanol, 6.6 g of hydroxylamine hydrochloride in 20 ml of water and 8.53 g of sodium acetate in 20 ml of water is refluxed

overnight. The product crystallizes on cooling. Recrystallization from 95% ethanol gives crystals, mp 105—109°C, of 1-(2-fluorophenyl)-2-(2-nitrophenyl)ethanone oxime.

Analysis:
Calculated for $C_{14}H_{11}FN_2O_3$: 61.31%C; 4.04%H; 10.21%N.
Found: 61.19%C; 4.09%H; 10.25%N.

Example 21

1-(2-Fluorophenyl)-2-(2-nitrophenyl)ethanone oxime acetate (V)

To a solution of 8 g of 1-(2-fluorophenyl)-2-(2-nitrophenyl)ethanone oxime in 20 ml of pyridine, 10 ml of acetic anhydride is added dropwise. The mixture is warmed (steam bath) for 3 1/2 hours, poured into water and extracted with methylene chloride. The methylene chloride extract is washed with 5% hydrochloric acid, dried over anhydrous sodium sulfate, filtered and evaporated. The residual oil is distilled at 5 mm to give 1-(2-fluorophenyl)-2-(2-nitrophenyl)ethanone oxime acetate, bp 133—135°C.

Analysis:
Calculated for $C_{16}H_{13}FN_2O_4$: 60.76%C; 4.14%H; 8.86%N.
Found: 60.83%C; 4.09%H; 9.11%N.

Example 22

α-(4-methoxyphenyl)-2-nitrophenethylamine hydrochloride (VI)

A stirred ice water chilled solution of 27.8 g of 1-(4-methoxyphenyl)-2-(2-nitrophenyl)ethanone oxime acetate and 250 ml of tetrahydrofuran is treated over 30 minutes with 360 ml of 0.94 M borane in tetrahydrofuran (four-fold excess of boron hydride). After total addition, the solution is stirred for 1 hour at ice-bath temperature, followed by stirring at ambient temperature for 4 hours. The solution is then allowed to stand for 2 days at ambient temperature. The stirred solution is chilled with an ice water bath and quenched by dropwise addition of 100 ml of 5% hydrochloric acid. A colorless precipitate separates and the mixture is stirred for 2 hours with cooling. Glacial acetic acid (15 ml) is added, followed by stirring for 2 hours and then standing overnight at ambient temperature. The stirred suspension is then treated with 300 ml of 10% sodium hydroxide solution, followed by removal of excess tetrahydrofuran on a rotary evaporator. The residual biphasic mixture is extracted thrice with 200 ml-portions of methylene chloride. The combined organic phase is dried over anhydrous sodium sulfate, vacuum filtered and evaporated to an orange colored oil. A solution of the oil and 200 ml of anhydrous ether is treated with a slight excess of ethereal hydrogen chloride. The precipitate is collected by vacuum filtration. The filter cake is washed twice with ether and dried *in vacuo* at 40°C over sodium hydroxide pellets. Recrystallization of the crude product from 600 ml of 95% ethanol gives 16.1 g of slightly yellow crystals, mp. 240—242°C, of α-(4-methoxyphenyl)-2-nitrophenethylamine hydrochloride.

Analysis:
Calculated for $C_{15}H_{16}N_2O_3{\cdot}HCl$: 58.35%C; 5.55%H.
Found: 58.21%C; 5.25%H.

Example 23

α-(4-methylphenyl)-2-nitrophenethylamine hydrochloride (VI)

To a cooled solution of 48 g of 1-(4-methylphenyl)-2-(2-nitrophenyl)ethanone oxime acetate in 350 ml of tetrahydrofuran in an atmosphere of nitrogen, 594 ml 1.01 M borane in tetrahydrofuran is added dropwise. The internal temperature remains approximately 0—20°C during the addition. The mixture is stirred for 45 minutes at ice bath temperature and then stirred overnight at ambient temperature. The mixture is cooled and the complex is decomposed by cautious addition of 6N hydrochloric acid. A precipitate gradually forms and the mixture is stirred overnight. Tetrahydrofuran is evaporated *in vacuum* and 10% sodium hydroxide solution is added to the residue. The product is extracted with ether and dried over anhydrous sodium sulfate. Ethereal hydrogen chloride is added to the solution and the product precipitates as the hydrochloride salt. Recrystallization from ethanol gives crystals, mp 265—269°C, of α-(4-methylphenyl)-2-nitrophenethylamine hydrochloride.

Analysis:
Calculated for $C_{15}H_{16}N_2O_2{\cdot}HCl$: 61.54%C; 5.85%H; 9.57%N.
Found: 61.57%C; 6.00%H; 9.44%N.

Example 24

α-(3,4-Dimethoxyphenyl)-2-nitrophenethylamine hydrochloride (VI)

To a cooled mixture of 92.6 g of 1-(3,4-dimethoxyphenyl)-2-(2-nitrophenyl)ethanone oxime acetate in 600 ml of tetrahydrofuran in an atmosphere of nitrogen, a solution of 1019 ml of 0.98 M borane in tetrahydrofuran is added dropwise. The mixture is allowed to stand overnight at ambient temperature. The mixture is cooled and 250 ml of 5% hydrochloric acid is added dropwise. The solvent is evaporated *in*

*vacuo* and the residue treated with 10% sodium hydroxide solution. The free amine is extracted with ether, dried over anhydrous sodium sulfate and filtered. Ethereal hydrogen chloride is added dropwise to the filtrate and the product precipitates as the hydrochloride salt. Recrystallization from methanol-ether gives crystals, mp 229—231°C, of α-(3,4-dimethoxyphenyl)-2-nitrophenethylamine hydrochloride.

Analysis:

| | | | |
|---|---|---|---|
| Calculated for $C_{16}H_{18}N_2O_4 \cdot HCl$: | 56.73%C; | 5.65%H; | 8.27%N. |
| Found: | 56.63%C; | 5.65%H; | 8.22%N. |

Example 25

α-(4-Fluorophenyl)-2-nitrophenethylamine hydrochloride (VI)

In an atmosphere of nitrogen, 897 ml of 0.98 M borane in tetrahydrofuran is added dropwise to a cooled solution of 69.2 g of 1-(4-fluorophenyl)-2-(2-nitrophenyl)ethanone oxime acetate in 500 ml of tetrahydrofuran. The mixture is allowed to stand over the weekend. The mixture is cooled and 200 ml of 5% hydrochloric acid is added dropwise. The tetrahydrofuran is evaporated and the residue treated with 10% sodium hydroxide solution. The free amine is extracted with ether and dried over anhydrous sodium sulfate. The ether solution is treated with ethereal hydrogen chloride and the product is recrystallized from ethanol to give crystals, mp 239—243°C, of α-(4-fluorophenyl)-2-nitrophenethylamine hydrochloride.

Analysis:

| | | | |
|---|---|---|---|
| Calculated for $C_{14}H_{13}FN_2O_2 \cdot HCl$: | 56.67%C; | 4.76%H; | 9.44%N. |
| Found: | 56.86%C; | 4.70%H; | 9.36%N. |

Example 26

N-Ethyl-2-nitro-α-phenylphenethylamine hydrochloride (VIII)

A 1 M solution of borane-tetrahydrofuran complex in tetrahydrofuran (42 ml) is added dropwise to a stirred cooled suspension of 6.00 g of N-acetyl-2-nitro-α-phenylphenethylamine in 60 ml of tetrahydrofuran. After complete addition the mixture is allowed to stir at room temperature for 24 hours, after which the excess borane is decomposed by the subsequent additions of 20 ml of 5% hydrochloric acid and 2 ml of acetic acid. The mixture is then basified with 20 ml of 50% sodium hydroxide solution. The tetrahydrofuran is evaporated from the biphasic mixture and the remaining aqueous phase is poured onto 100 ml of water. The aqueous mixture is extracted with methylene chloride (2 × 100 ml). The combined methylene chloride extracts are dried over anhydrous magnesium sulfate and evaporated to afford a yellow oil which is dissolved in 50 ml of ether. Ethereal hydrogen chloride (100 ml) is added precipitating the salt as an oil. The ether is evaporated and the remaining oil is dissolved in 20 ml of 100% ethanol and allowed to crystallize to provide crystals, mp 216—225°C dec, of N-ethyl-2-nitro-α-phenylphenethylamine hydrochloride.

Analysis:

| | | | |
|---|---|---|---|
| Calculated for $C_{16}H_{18}N_2O_2 \cdot HCl$: | 62.64%C; | 6.24%H; | 9.13%N. |
| Found: | 62.53%C; | 6.20%H; | 9.22%N. |

Example 27

2-Nitro-α-phenyl-N-propylphenethylamine hydrochloride (VIII)

1 M borane-tetrahydrofuran complex (42 ml) is added dropwise to a cooled, stirred suspension of 6.00 g of 2-nitro-α-phenyl-N-propionylphenethylamine in 60 ml of tetrahydrofuran. After complete addition, the mixture is allowed to stir at room temperature for 24 hours after which the excess borane is decomposed by the successive additions of 20 ml of 5% hydrochloric acid and 2 ml of acetic acid. The acidic mixture is allowed to stir an additional 30 minutes, after which the mixture is basified with 20 ml of 50% sodium hydroxide solution. The mixture is then poured onto 100 ml of water and the aqueous phase extracted with methylene chloride (2 × 100 ml). The combined methylene chloride extracts are dried over anhydrous magnesium sulfate and the methylene chloride evaporated affording a yellow oil, which is dissolved in 50 ml of ether. Ethereal hydrogen chloride (100 ml) is added precipitating the hydrochloride salt as an oil. The ether is evaporated and the resulting gum is dissolved in 40 ml of hot isopropanol. Upon cooling white crystals, mp 189—192°C, of 2-nitro-α-phenyl-N-propylphenethylamine hydrochloride falls from solution.

Analysis:

| | | | |
|---|---|---|---|
| Calculated for $C_{17}H_{20}N_2O_2 \cdot HCl$: | 63.65%C; | 6.60%H; | 8.73%N. |
| Found: | 63.64%C; | 6.88%H; | 8.38%N. |

Example 28

N-Benzyl-2-nitro-α-phenylphenethylamine hydrochloride (VIII)

1 M Borane-tetrahydrofuran complex (60 ml) is added dropwise to a cooled, stirred solution of 9.00 g of N-benzoyl-2-nitro-α-phenylphenethylamine in 90 ml of tetrahydrofuran. After complete addition the

mixture is stirred at room temperature for 4 hours. Additional tetrahydrofuran (100 ml) and 1 M borane-tetrahydrofuran complex (30 ml) are added to aid in the dissolution of the reactants. The mixture is allowed to stir at room temperature for 24 hours after which the excess borane is decomposed by the subsequent addition of 45 ml of 5% hydrochloric acid and 5 ml of acetic acid. The acidic mixture is stirred at room temperature for an additional 0.5 hours after which 25 ml of 50% sodium hydroxide solution is added. The tetrahydrofuran is evaporated from the mixture and the resulting aqueous mixture is poured onto 125 ml of water. The aqueous phase is extracted with methylene chloride (1 × 150 ml, 1 × 100 ml). The combined methylene chloride extracts are dried over anhydrous magnesium sulfate and evaporated to give a solid which recrystallizes from 150 ml of 100% ethanol giving floculIant crystals. The ethanolic filtrate is acidified with 100 ml of ethereal hydrogen chloride. Upon prolonged standing the hydrochloride salt falls from solution. Recrystallization of the salt from 40 ml of ethanol gives yellow granules, mp 215—218°C, of N-benzyl-2-nitro-α-phenylphenethylamine hydrochloride.

Analysis:

| | | | |
|---|---|---|---|
| Calculated for $C_{21}H_{20}N_2O_2 \cdot HCl$: | 68.38%C; | 5.74%H; | 7.59%N. |
| Found: | 68.11%C; | 5.58%H; | 7.66%N. |

Example 29

N-Cyclohexylmethylene-2-nitro-α-phenylphenethylamine hydrochloride (VIII)

1 M Borane-tetrahydrofuran complex (60 ml) is added dropwise to a stirred, cooled suspension of 9.00 g of N-cyclohexylcarbonyl-2-nitro-α-phenylphenethylamine in 90 ml of tetrahydrofuran. After complete addition the mixture is allowed to stir for 4 hours. Additional borane (30 ml) and tetrahydrofuran (100 ml) is added to complete dissolution of materials. The mixture is stirred an additional 16 hours. After cooling of the mixture, the excess borane is decomposed by the subsequent additions of 45 ml of 5% hydrochloric acid and 4 ml of acetic acid. The mixture is stirred for 1/2 hour before the addition of 25 ml of 50% sodium hydroxide solution. The tetrahydrofuran is evaporated from the mixture and the resulting biphasic mixture is poured onto 125 ml of water. The aqueous mixture is extracted with methylene chloride (1 × 150 ml, 1 × 100 ml). The combined methylene chloride extracts are dried over anhydrous magnesium sulfate and the methylene chloride is evaporated giving a yellow oil. The oil is dissolved in 100 ml of ether. Ethereal hydrogen chloride (200 ml) is added. Upon standing, a white precipitate falls from solution. The ether is evaporated and the solid is recrystallized from 50 ml of ethanol giving crystals, mp 225—228°C, of N-cyclohexylmethylene-2-nitro-α-phenylphenethylamine hydrochloride.

Analysis:

| | | | |
|---|---|---|---|
| Calculated for $C_{21}H_{26}N_2O_2 \cdot HCl$: | 67.28%C; | 7.26%H; | 7.47%N. |
| Found: | 67.08%C; | 7.26%H; | 7.51%N. |

Example 30

α-(4-Methoxyphenyl)-N-methyl-2-nitrophenethylamine hydrochloride (VIII)

A stirred ice water chilled suspension of 12.3 g of N-formyl-α-(4-methoxyphenyl)-2-nitrophenethylamine and 120 ml of tetrahydrofuran is treated over 20 minutes with 85 ml of 1.01 M borane in tetrahydrofuran. After total addition the solution is stirred for 3 hours with ice water cooling and then allowed to stand 2 days at ambient temperature with exclusion of moisture. The stirred solution is chilled and quenched by dropwise addition of 40 ml of 5% hydrochloric acid and 4 ml of glacial acetic acid. Two hours after total addition the mixture is made alkaline with 50% sodium hydroxide solution, diluted with 50 ml of water and concentrated on a rotary evaporator to remove the tetrahydrofuran. The residual liquid is extracted thrice with 70 ml portions of dichloromethane and the combined organic phase is dried over anhydrous sodium sulfate, filtered and concentrated to an oil. A solution of the oil and 100 ml of ether is treated with ethereal hydrogen chloride giving a gum. The ether-gum mixture is evaporated to a yellow colored amorphorous foam which is recrystallized from isopropanol to provide almost colorless crystals, mp 181—185°C, of α-(4-methoxyphenyl)-N-methyl-2-nitrophenethylamine hydrochloride.

Analysis:

| | | | |
|---|---|---|---|
| Calculated for $C_{16}H_{18}N_2O_3 \cdot HCl$: | 59.54%C; | 5.93%H; | 8.68%N. |
| Found: | 59.24%C; | 5.81%H; | 8.65%N. |

Example 31

α-(3,4-Dimethoxyphenyl)-N-methyl-2-nitrophenethylamine hydrochloride (VIII)

To a cooled mixture of 33.6 g of N-formyl-α-(4-methoxyphenyl)-2-nitrophenethylamine in 500 ml of tetrahydrofuran, 408 ml of 0.98 M of boron hydride/tetrahydrofuran is added dropwise keeping the internal temperature at 0—10°C. The mixture is allowed to stand overnight at ambient temperature. To the cooled mixture 100 ml of 5% hydrochloric acid is added cautiously, followed by addition of 20 ml of glacial acetic acid. The solvent is evaporated *in vacuo* and the residue treated with 10% sodium hydroxide solution. The free base is extracted with ether, dried over anhydrous sodium sulfate and filtered. The product is precipitated as the hydrochloride salt by addition of ethereal hydrogen chloride to the ether extract.

Recrystallization from ethanol gives crystals, mp 185—187.5°C, of α-(3,4-dimethoxyphenyl)-N-methyl-2-nitrophenethylamine hydrochloride.

Analysis:

| | | | |
|---|---|---|---|
| Calculated for $C_{17}H_{20}N_2O_4 \cdot HCl$: | 57.87%C; | 6.00%H; | 7.94%N. |
| Found: | 57.77%C; | 6.09%H; | 7.93%N. |

Example 32

α-(4-Fluorophenyl)-N-methyl-2-nitrophenethylamine hydrochloride (VIII)

To a cooled mixture of 31.1 g of N-formyl-α-(4-fluorophenyl)-2-nitrophenethylamine in 500 ml of tetrahydrofuran, 441 ml of 0.98 M borane in tetrahydrofuran is added dropwise. The mixture is allowed to stand over the weekend. The mixture is cooled and 100 ml of 5% hydrochloric acid and 20 ml of glacial acetic acid are added cautiously. The solvent is evaporated *in vacuum* and the residue treated with 10% sodium hydroxide solution. The free amine is extracted with ether and dried over anhydrous sodium sulfate and filtered. Ethereal hydrogen chloride is added dropwise to the filtrate and the product precipitates as the hydrochloride salt. Recrystallization from ethanol gives crystals, mp 241—243°C, of α-(4-fluorophenyl)-N-methyl-2-nitrophenethylamine hydrochloride.

Analysis:

| | | | |
|---|---|---|---|
| Calculated for $C_{15}H_{15}FN_2O_2 \cdot HCl$: | 57.98%C; | 4.87%H; | 9.01%N. |
| Found: | 57.89%C; | 5.15%H; | 9.01%N. |

Example 33

α-(4-Methylphenyl)-N-methyl-2-nitrophenethylamine hydrochloride (VIII)

To a cooled solution of 17 g of N-formyl-α-(4-methylphenyl)-2-nitrophenethylamine in 200 ml of tetrahydrofuran, 122 ml of 0.98 M borane in tetrahydrofuran is added dropwise in an atmosphere of nitrogen. The temperature did not exceed 10°C during the addition. The mixture is allowed to stir at room temperature for 4 hours. Excess borane is decomposed by addition of 50 ml of 5% hydrochloric acid and 10 ml of acetic acid. The mixture is allowed to stand over the weekend. Tetrahydrofuran is evaporated *in vacuo*. The residue is treated with 10% sodium hydroxide solution, extracted with ether and the organic phase is dried over anhydrous sodium sulfate and filtered. Ethereal hydrogen chloride is added to the filtrate and the hydrochloride salt precipitates. Recrystallization from ethanol yields crystals, mp 235—238°C, of α-(4-methylphenyl)-N-methyl-2-nitrophenethylamine hydrochloride.

Analysis:

| | | | |
|---|---|---|---|
| Calculated for $C_{16}H_{18}N_2O_2 \cdot HCl$: | 62.64%C; | 6.24%H; | 9.13%N. |
| Found: | 62.50%C; | 6.23%H; | 9.14%N. |

Example 34

N-Acetyl-2-nitro-α-phenylphenethylamine (VII)

10.0 g of N-Methyl-2-nitro-α-phenylphenethylamine in 75 ml of toluene is added in a dropwise manner to a cooled, stirred solution of 10.2 ml of acetic anhydride in 100 ml of toluene. Upon completion of addition, the mixture is allowed to remain at room temperature for 72 hours, after which the precipitate is collected, washed with ether (2 × 50 ml) and dried. Recrystallization from 100% ethanol gives crystals, mp 171—172°C, of N-acetyl-2-nitro-α-phenylphenethylamine.

Analysis:

| | | | |
|---|---|---|---|
| Calculated for $C_{16}H_{16}N_2O_3$: | 67.59%C; | 5.67%H; | 9.85%N. |
| Found: | 67.50%C; | 5.61%H; | 9.95%N. |

Example 35

N-Propionyl-2-nitro-α-phenylphenethylamine (VII)

10.0 g of N-methyl-2-nitro-α-phenylphenethylamine in 75 ml of toluene is added in a dropwise manner to a cooled, stirred solution of 10.5 ml of propionic anhydride in 100 ml of toluene. Upon completion of addition, the mixture is allowed to remain at room temperature for 75 hours, after which the precipitate is collected, washed with ether (2 × 50 ml) and dried to give white powder. Recrystallization from 100% ethanol ÷ water &0 ml ÷ 30 ml) gives pale yellow needles, mp 149—151°C, of N-propionyl-2-nitro-α-phenylphenethylamine.

Analysis:

| | | | |
|---|---|---|---|
| Calculated for $C_{17}H_{18}N_2O_3$: | 68.44%C; | 6.08%H; | 9.39%N. |
| Found: | 68.30%C; | 5.94%H; | 9.26%N. |

Example 36

N-Benzoyl-2-nitro-α-phenylphenethylamine (VII)

10.0 g of 2-nitro-α-phenylphenethylamine in 75 ml of toluene is added dropwise to a stirred, cooled

solution of 24.3 g of benzoic anhydride in 75 ml of toluene. After complete addition, the mixture is stirred at room temperature for 2 hours after which the precipitate is collected, washed with 300 ml of ether and partially dried. The precipitate is heated in boiling 400 ml ethanol, cooled and collected giving white needles, mp 185—189°C, of N-benzoyl-2-nitro-α-phenylphenethylamine.

Analysis:

| | | | |
|---|---|---|---|
| Calculated for $C_{21}H_{18}N_2O_2$: | 72.82%C; | 5.24%H; | 8.09%N. |
| Found: | 72.54%C; | 5.29%H; | 8.07%N. |

Example 37

N-Cyclohexanecarbonyl-2-nitro-α-phenylphenethylamine (VII)

10.0 g of 2-nitro-α-phenylphenethylamine in 75 ml of toluene is added dropwise to a stirred, cooled solution of 14.4 ml of cyclohexylcarbonyl chloride in 75 ml of toluene and 25 ml of pyridine. After complete addition the mixture is stirred at room temperature for 2 hours. The mixture is allowed to stand overnight, after which the tan precipitate is collected and washed with 100 ml of ether. The precipitate is recrystallized from 350 ml of methanol to give yellow needles, mp 210—212°C, of N-cyclohexanecarbonyl-2-nitro-α-phenylphenethylamine.

Analysis:

| | | | |
|---|---|---|---|
| Calculated for $C_{21}H_{24}N_2O_3$: | 71.57%C; | 6.86%H; | 7.95%N. |
| Found: | 71.36%C; | 6.92%H; | 7.99%N. |

Example 38

N-Formyl-α-(4-methoxyphenyl)-2-nitrophenethylamine (VII)

A suspension of 14.0 g of α-(4-methoxyphenyl)-2-nitrophenethylamine hydrochloride, and 250 ml of water is treated with 70 ml of 10% sodium hydroxide solution. The mixture is twice extracted with 200 ml-portions of methylene chloride and the combined organic phase is dried over anhydrous sodium sulfate. Vacuum filtration and concentration affords 12.7 g of a yellow-colored oil. A solution of the oil and 200 ml of methyl formate is heated at 80—83°C (bath temperature) for 3 days in a 300 ml Parr stainless steel bomb. After cooling to room temperature the bomb is opened and the crystalline precipitate is collected by vacuum filtration. The filter cake is washed once with methylformate and dried *in vacuo* at 40°C to give slightly yellow-colored crystals, mp 151—153°C, of N-formyl-α-(4-methoxyphenyl)-2-nitrophenethylamine.

Analysis:

| | | | |
|---|---|---|---|
| Calculated for $C_{16}H_{16}N_2O_4$: | 63.99%C; | 5.37%H; | 9.33%N. |
| Found: | 63.92%C; | 5.29%H; | 9.37%N. |

Example 39

N-Formyl-α-(4-methylphenyl)-2-nitrophenethylamine (VII)

A mixture of 19.6 g of α-(4-methylphenyl)-2-nitrophenethylamine and 230 ml of methylformate is placed in a Paar bomb at 80°C overnight. The reaction is cooled and the solvent removed *in vacuo* giving an off-white solid. The product is recrystallized from 95% ethanol giving crystals, mp 130—132°C, of N-formyl-α-(4-methylphenyl)-2-nitrophenethylamine.

Analysis:

| | | | |
|---|---|---|---|
| Calculated for $C_{16}H_{16}N_2O_3$: | 67.59%C; | 5.67%H; | 9.85%N. |
| Found: | 67.58%C; | 5.63%H; | 9.94%N. |

Example 40

N-Formyl-α-(4-fluorophenyl)-2-nitrophenethylamine (VII)

A mixture of 36.6 g of α-(4-fluorophenyl)-2-nitrophenethylamine and 230 ml of methylformate in a Parr bomb is placed in an oil bath at 80°C and left over the weekend. On cooling a crystalline precipitate separates giving 34.6 g of product. An analytical sample is recrystallized from 95% ethanol providing crystals, mp 142—145°C, of N-formyl-α-(4-fluorophenyl)-2-nitrophenethylamine.

Analysis:

| | | | |
|---|---|---|---|
| Calculated for $C_{15}H_{13}FN_2O_3$: | 62.50%C; | 4.54%H; | 9.72%N. |
| Found: | 62.62%C; | 4.51%H; | 9.77%N. |

Example 41

N-Formyl-α-(3,4-dimethoxyphenyl)-2-nitrophenethylamine (VII)

A mixture of 41.7 g of α-(3,4-dimethoxyphenyl)-2-nitrophenethylamine and 250 ml of methylformate is placed in a Parr bomb at 80°C and left overnight. The solvent is evaporated and the residue recrystallized

from 95% ethanol giving crystals, mp 135—139°C, of N-formyl-α-(3,4-dimethoxyphenyl)-2-nitrophenethylamine.

Analysis:
Calculated for $C_{17}H_{18}N_2O_5$: 61.81%C; 5.49%H; 8.48%N.
Found: 61.67%C; 5.27%H; 8.42%N.

Example 42

N-Acetyl-α-(3,4-dimethoxyphenyl)-N-methyl-2-nitrophenethylamine (VII)

To a cooled solution of 4.17 g of acetic anhydride in 50 ml of toluene, a solution of 4.31 g of α-(3,4-dimethoxyphenyl)-N-methyl-2-nitrophenethylamine is added dropwise. The mixture is warmed to room temperature and stirred for 2 hours. The solvents are evaporated *in vacuo* to give crystals, mp 113—115°C, of N-acetyl-α(3,4-dimethoxyphenyl)-N-methyl-2-nitrophenethylamine.

Analysis:
Calculated for $C_{19}H_{22}N_2O_5$: 63.67%C; 6.18%H; 7.82%N.
Found: 63.68%C; 6.20%H; 7.76%N.

**Claims**

1. A compound of the formula II

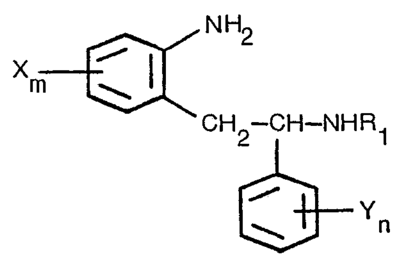

II

in which $R_1$ is hydrogen, alkyl of from 1 to 3 carbon atoms, cycloalkyl-$C_1$—$C_3$-alkyl wherein the cycloalkyl radial has 3 to 6 carbon atoms, or aralkyl having up to 8 carbon atoms, X and Y are the same or different and each can be chlorine, bromine, fluorine, alkoxy or alkyl each of from 1 to 3 carbon atoms, hydroxy, or trifluoromethyl; m is the integer 0, 1 or 2; and n is the integer 0, 1, 2 or 3.

2. A method for preparing a compound of the formula II

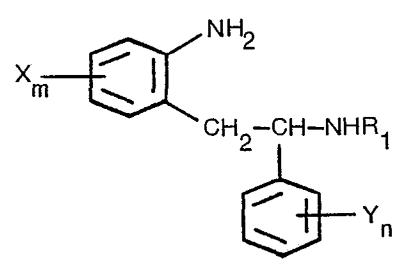

II

in which $R_1$ is hydrogen, alkyl of from 1 to 3 carbon atoms, cycloalkyl-$C_1$—$C_3$-alkyl wherein the cycloalkyl radical has 3 to 6 carbon atoms, or aralkyl having up to 8 carbon atoms, X and Y are the same or different and each can be chlorine, bromine, fluorine, alkoxy or alkyl each of from 1 to 3 carbon atoms, hydroxy, or trifluoromethyl; m is the integer 0, 1 or 2; and n is the integer 0, 1, 2 or 3, which comprises reducing a compound of the formula VI

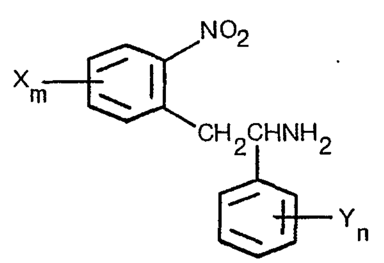

VI

or a compound of the formula VIII

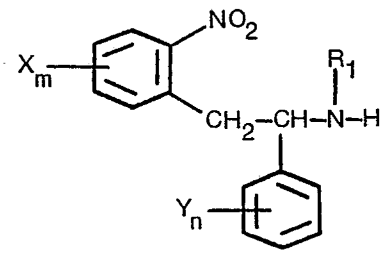

VIII

wherein $R_1$, X, Y, m and n are as defined in claim 1.

3. The method defined in claim 2 in which the reduction is carried through by hydrogenation with a palladium on carbon catalyst or Adam's catalyst.

**Patentansprüche**

1. Verbindungen der Formel II

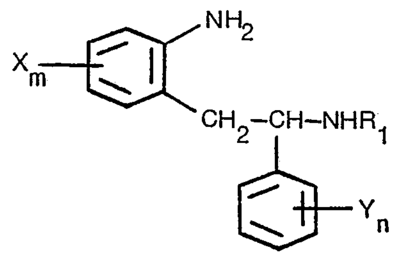

II

worin

$R_1$ Wasserstoff, Alkyl mit 1—3 Kohlenstoffatomen, Cycloalkyl-$C_1$—$C_3$-alkyl, in welchem das Cycloalkylradikal 3—6 Kohlenstoffatome aufweist oder Aralkyl mit bis zu 8 Kohlenstoffatomen bedeutet,

X und Y gleich oder verschieden sind und jedes für sich Chlor, Brom, Fluor, Alkoxy oder Alkyl mit jeweils 1—3 Kohlenstoffatomen, Hydroxy oder Trifluormethyl bedeuten kann;

m eine ganze Zahl 0, 1 oder 2 und

n eine ganze Zahl 0, 1, 2 oder 3 bedeuten.

2. Verfahren zur Herstellung einer Verbindung der Formel II

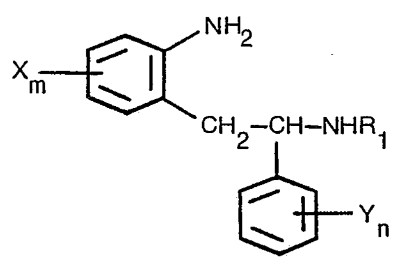

II

worin

$R_1$ Wasserstoff, Alkyl mit 1—3 Kohlenstoffatomen, Cycloalkyl-$C_1$—$C_3$-alkyl, in welchem das Cycloalkylradikal 3—6 Kohlenstoffatome aufweist oder Aralkyl mit bis zu 8 Kohlenstoffatomen bedeutet,

X und Y gleich oder verschieden sind und jedes für sich Chlor, Brom, Fluor, Alkoxy oder Alkyl mit jeweils 1—3 Kohlenstoffatomen, Hydroxy oder Trifluormethyl bedeuten kann;

m eine ganze Zahl 0, 1 oder 2 und

n eine ganze Zahl 0, 1, 2 oder 3 bedeuten, gekennzeichnet durch die Reduktion einer Verbindung der Formel VI

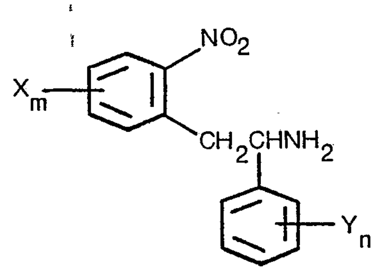

VI

oder einer Verbindung der Formel VIII

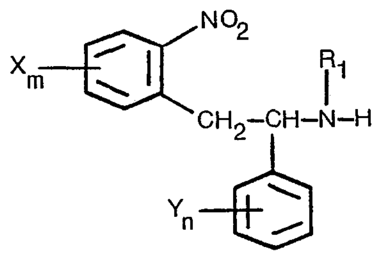

VIII

worin

$R_1$, X, Y, m und n jeweils wie in Anspruch 1 definiert sind.

3. Verfahren nach Anspruch 2, dadurch gekennzeichnet, dass die Reduktion durch Hydrierung mittels Palladium auf Aktivkohle- oder Adams-Katalysator durchgeführt wird.

**Revendications**

1. Composé de formule II:

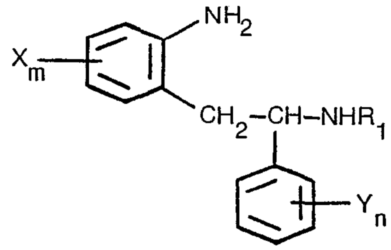

II

dans laquelle $R_1$ est l'hydrogène, un groupe alcoyle ayant de 1 à 3 atomes de carbone, cycloalcoyl-alcoyle($C_1$—$C_3$) dans lequel le groupe cycloalcoyle a de 3 à 6 atomes de carbone, ou aralcoyle ayant jusqu'à 8 atomes de carbone, X et Y sont identiques ou différents et peuvent représenter chacun le chlore, le brome, le fluor, un groupe alcoxy ou alcoyle ayant chacun de 1 à 3 atomes de carbone, hydroxy ou trifluorométhyle; m est le nombre 0, 1 ou 2; et n est le nombre 0, 1, 2 ou 3.

2. Procédé de préparation d'un composé de formule II:

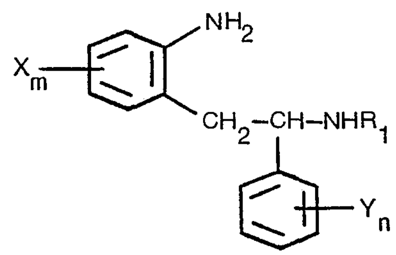

II

dans laquelle $R_1$ est l'hydrogène, un groupe alcoyle ayant de 1 à 3 atomes de carbone, cycloalcoyl-alcoyle($C_1$—$C_3$) dans lequel le groupe cycloalcoyle a de 3 à 6 atomes de carbone ou aralcoyle ayant jusqu'à 8 atomes de carbone, X et Y sont identiques ou différents et peuvent représenter chacun le chlore, le brome, le fluor un groupe alcoxy ou alcoyle ayant chacun de 1 à 3 atomes de carbone, un groupe hydroxy ou trifluorométhyle; m est le nombre 0, 1 ou 2; et n est le nombre 0, 1, 2 ou 3, qui consiste à réduire un composé de formule VI:

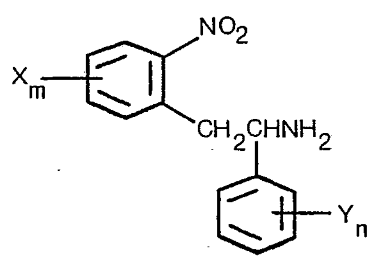

VI

ou un composé de formule VIII:

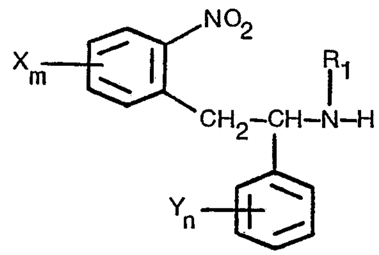

VIII

dans lesquelles $R_1$, X, Y, m et n sont tels que définis dans la revendication 1.

3. Procédé selon la revendication 2, dans lequel la réduction est effectuée par hydrogénation avec un catalyseur au palladium sur carbone ou un catalyseur d'Adam.